# EUROPÄISCHE PATENTSCHRIFT

(11) **EP 0 963 399 B1**
(45) Veröffentlichungstag und Bekanntmachung des Hinweises auf die Patenterteilung: **14.04.2004**
(21) Anmeldenummer: 98905265.9
(22) Anmeldetag: 14.01.1998
(51) Int. Cl.: C08H 5/02

(54) **ZWISCHENPRODUKT FÜR DIE HERSTELLUNG VON LIGNINPOLYMERISATEN UND DESSEN VERWENDUNG FÜR DIE HERSTELLUNG VON REAGENTIEN FÜR HERSTELLUNG VON VERBUNDWERKSTOFFEN AUS PFLANZLICHEN FASERN, WASSERFESTEN PAPIEREN UND PAPPEN SOWIE DUROPLASTEN AUS LIGNINDERIVATEN**
INTERMEDIATE FOR THE PRODUCTION OF LIGNIN POLYMERS AND THEIR USE IN THE PRODUCTION OF REAGENTS FOR THE PRODUCTION OF COMPOSITE MATERIALS FROM PLANT FIBERS, WATERPROOF PAPERS AND CARDBOARDS AS WELL AS DUROPLASTICS FROM LIGNIN DERIVATIVES
PRODUIT INTERMEDIAIRE POUR LA PRODUCTION DE POLYMERES DE LIGNINE, ET SON UTILISATION POUR LA FABRICATION DE REACTIFS POUR LA PRODUCTION DE COMPOSITES DE FIBRES VEGETALES, DE PAPIERS ET CARTONS IMPERMEABILISES, AINSI QUE DE DUROPLASTES A BASE DE DERIVES DE LIGNINE

(30) Priorität: 14.01.1997 DE 19700902; 14.01.1997 DE 19700904; 14.01.1997 DE 19700906
(43) Veröffentlichungstag der Anmeldung: 15.12.1999
(73) Patentinhaber: STOCKHAUSEN GmbH & Co. KG, 47805 Krefeld (DE)
(72) Erfinder: HÜTTERMANN, Aloys, D-37075 Göttingen (DE); MAJCHERCZYK, Andrzej, D-37077 Göttingen (DE); MAI, Carsten, D-37085 Göttingen (DE); BRAUN-LÜLLEMANN, Annette, D-37120 Bovenden (DE); FASTENRATH, Merle, D-37077 Göttingen (DE); NOETZOLD, Sonja, D-37077 Göttingen (DE)
(74) Vertreter: Harders, Gerhard, Dr.
(86) Internationale Anmeldenummer: PCT/DE1998/000254
(87) Internationale Veröffentlichungsnummer: WO 1998/031728

(56) Entgegenhaltungen:
- EP-A- 0 919 628
- WO-A-93/23477
- WO-A-94/01488
- DE-A- 3 037 992
- US-A- 5 110 414

## Beschreibung

Die vorliegende Erfindung betrifft ein Zwischenprodukt für die Herstellung von Polymerisaten aus Ligninderivaten, die bei der Zellstoffproduktion anfallen, sowie die Verwendung dieser Zwischenprodukte bei der Herstellung von hochaktiven Reagentien für die Herstellung von Verbundwerkstoffen aus pflanzlichen Fasern, wasserfesten Papieren und Pappen sowie Duroplasten aus Ligninderivaten.

In der DE 3037992 ist ein Verfahren zur Herstellung eines Bindemittels für Holzwerkstoffe unter Verwendung von phenolischen Stoffen, insbesondere Ligninsulphonat, beschrieben, bei dem zwecks Aktivierung der phenolische Stoff mit Enzymen versetzt wird, die Phenole nach einem Radikalmechanismus oxidativ polymerisieren, wobei der phenolische Stoff in ein aktives Bindemittel umgeformt wird. Es war zwar bekannt, daß diese Reaktion in Gegenwart von Sauerstoff, z.B. Luftsauerstoff, abläuft, aber bisher war ein solches aktiviertes Bindemittel nicht längere Zeit oder durch intensives Belüften mit Sauerstoff umgesetzt worden.

Überraschenderweise wurde gefunden, daß aus der Zellstoffindustrie stammende Ligninderivate, wie beispielsweise Kraftlignin oder Ligninsulfonat, mit dem phenoloxidierenden Enzym Laccase, bei intensiver oder langandauernder Umsetzung mit Sauerstoff, Luft oder anderen chemischen Oxidationsmitteln ein besonders reaktionsfähiges Ligninprodukt als Zwischenprodukt bildet. Dieses kann isoliert und längere Zeit gelagert werden und reagiert mit weiteren, nicht aktivierten Ligninderivaten zu einem hochmolekularen Polymerisat weiter. Das Zwischenprodukt kann dadurch charakterisiert werden, daß das Material mit Laccase umgesetzt wird. Es weist nach dieser Reaktion ein typisches ESR-Spektrum mit einem Signal für Phenoxyradikale im Bereich von ca. 3400 Gauss auf, welches aber als typisches Radikalsignal auf Dauer nicht beständig erhalten bleibt. Überraschenderweise bleibt aber die erhöhte Reaktivität des Zwischenprodukts auch nach langen Zeiträumen. wie beispielsweise nach Monaten, noch erhalten. Das heißt, daß dieses aktivierte Zwischenprodukt beim Umsatz mit phenoloxidierenden Enzymen erheblich aktiver ist als nicht vorbehandelte Ligninderivate und demgemäß das typische ESR-Spektrum in wesentlich höherer Intensität ausbildet als nicht derart vorbehandelte Ligninderivate.

Die Intensität des Signals des aktivierten Zwischenprodukts beträgt zumindest mehr als das Fünffache des Signals des als Ausgangsprodukt dienenden Ligninderivats. Das Signal wird beispielsweise unter folgenden Bedingungen gemessen:
77°K; 9,5 Ghz, ESR-Dämpfung 20 dB, Mod.-Freq. 100 Mhz; Mod. Amplitude 4,0 Gauss.

Das aktivierte Zwischenprodukt kann erhalten werden, indem technische Lignine, wie beispielsweise Ligninsulfonat, Kraftlignin, Organosolvlignin, Acetosolvlignin, ASAM-Lignin, die bei der Zellstoffproduktion anfallen, längere Zeit in Gegenwart von phenoloxidierenden Enzymen mit Luft oder Sauerstoff behandelt werden. Bereits nach einem Zeitraum von beispielsweise ca. drei Stunden, insbesondere aber nach 15 oder 20 Stunden, ist eine Erhöhung des Phenoxyradikal-Signals festzustellen. Beim Durchleiten von Luft oder Sauerstoff unter Druck tritt das erhöhte Signal bereits nach wesentlich kürzererZeitauf, nach 10 min oder beispielsweise ca. 30 min.

Die enzymatische Bildung des aktivierten Zwischenprodukts ist nur in Gegenwart größerer Mengen Sauerstoff möglich. Da sich bei Zimmertemperatur in Wasser nur 9 mg/l Sauerstoff lösen, wird die Bildung des Zwischenprodukts nur durch erhöhte Zufuhr von Sauerstoff, entweder durch Belüften oder durch Zugabe von Oxidationsmitteln, begünstigt. Auch wenn sich das Sauerstoff-Gleichgewicht über einen längeren Zeitraum einstellt, kann nach einiger Zeit genügend Sauerstoff auf das Ligninderivat eingewirkt haben.

Das aktivierte Zwischenprodukt reagiert in Gegenwart von phenoloxidierenden Enzymen beispielsweise mit nichtaktivierten Ligninderivaten, wie sie beispielsweise aus der Zellstoffproduktion gewonnen werden können, unter Ausbildung von polymeren Ligninprodukten, wobei die erhaltenen Molgewichte wesentlich höher sind als jene, die bei der Einwirkung von phenoloxidierenden Enzymen auf Ligninderivate ohne die Gegenwart aktivierter Ligninderivate erhalten werden.

Sie liegen im allgemeinen mindestens um das Doppelte höher.

Die bei der Polymerisation von Ligninderivaten in Gegenwart der aktiven Zwischenprodukte erhaltenen Ligninpolymere lassen sich für die Herstellung von hochaktiven Reagentien für die Herstellung von Verbundwerkstoffen aus pflanzlichen Fasern, wasserfesten Papieren und Pappen sowie Duroplasten aus Ligninderivaten. verwenden. Damit ist es erstmals möglich, faserverstärkte Duroplaste ganz durch *in situ* - Polymerisation aus nachwachsenden Rohstoffen herzustellen.

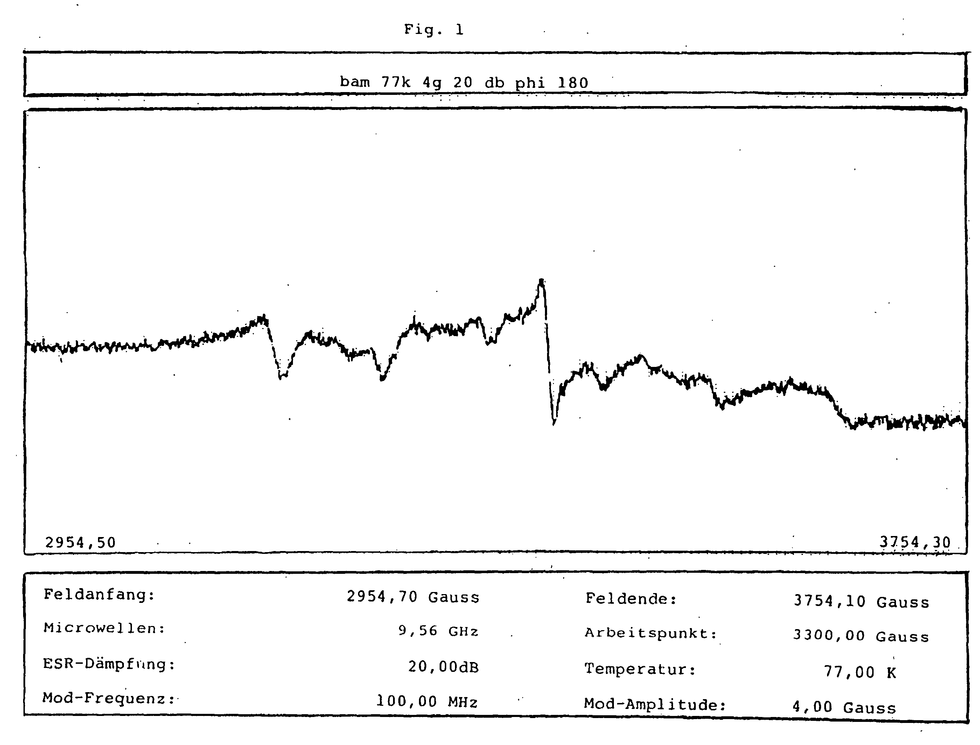

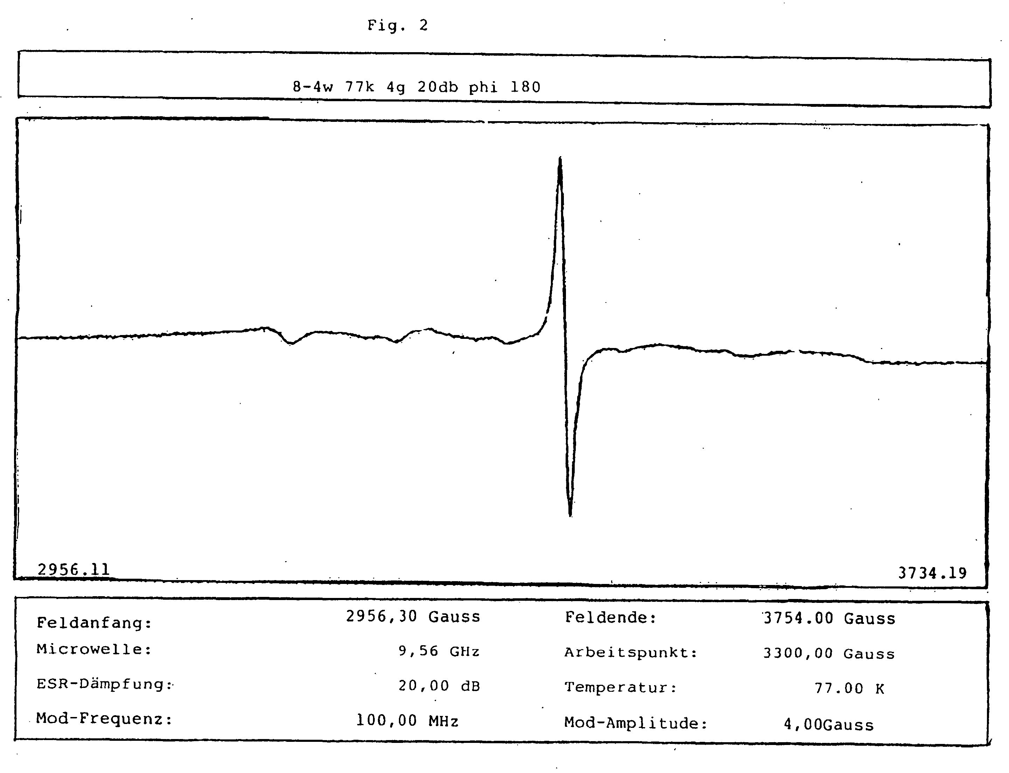

Das aktivierte Lignin weist gegenüber dem als Ausgangsmaterial verwendeten Lignin ein ESR-Spektrum mit wesentlich erhöhter Intensität des Phenoxyradikal-Signals auf. Dies wird durch die Figuren 1 und 2 dargestellt. Figur 1 zeigt ein ESR-Spektrum von 1 % Ligninsulfonat mit Zusatz von Laccase ( 4 U/ml nach 30 minütiger Inkubation ohne Sauerstoffbehandlung. Figur 2 zeigt das entsprechende Spektrum von Ligninsulfonat, das 20 Stunden unter erhöhter Sauerstoffzufuhr mit Laccase inkubiert und anschließend autoklaviert und drei Monate gelagert wurde. Nach erneuter Inkubation mit Laccase ( 4 U/ml, 30 min. Inkubation ohne Sauerstoffbehandlung) zeigt der Vergleich des stärksten Signals bei ca. 3400 Gauss mit den Hintergrundsignalen. daß gegenüber der Figur 1 die Intensität des Phenoxyradikal-Signals um mindestens das Fünffache verstärkt wurde.

Die Reaktivität des entstehenden Zwischenprodukts ist derart hoch, daß selbst Ligninsulfonat, ein Polymer mit einer äußerst hohen Wasserlöslichkeit, ein wasserunlösliches Produkt bildet.

Die Erfindung wird nachfolgend anhand der Beispiele näher erläutert:

### BEISPIEL 1

20 g Ligninsulfonat werden in 80 ml McIlvaine-Puffer, pH 5,5 gelöst und mit 800 U/ml Laccase versetzt. Die Lösung wird ein einem 500 ml Erlenmeyerkolben bei 37°C im Wasserbad für 20 Stunden geschüttelt. Die Lösung wird anschließend autoklaviert. Das erhaltene Ligninsulfonat wird zwei Monate gelagert. Nach erneuter Inkubation mit Laccase ( 4 U/ml, 30 min. Inkubation ohne Sauerstoffbehandlung) hat es das ESR-Spektrum gemäß Fig 2.

### BEISPIEL 2

Aktiviertes Ligninsulfonat nach Beispiel 1 wird im Verhältnis 1 : 10 mit Kraftlignin versetzt und mit einer Konzentration von 100 mg/10 ml in Puffer suspendiert und im verschlossenen Reagenzglas, ohne besondere Sauerstoffbehandlung, mit Laccase ( 500 U /ml) für 6 h inkubiert. Parallel dazu wurden entsprechende Kontrollversuche mit nichtaktiviertem Lginin und Inkubationen ohne Laccase durchgeführt. Die Lignine wurden anschließend isoliert und die Molekulargewichtsverteilung in der HPLC gemessen.

Dabei wurden die folgenden Molekulargewichte ermittelt:

| | |
|---|---|
| nichtaktiviertes Kraftlignin | 5.400 g/Mol |
| nichtaktiviertes Kraftlignin inkubiert mit Laccase | 6.300 g/Mol |
| nichtaktiviertes Kraftlignin plus aktiviertes Lignin ohne Laccase | 6.000 g/Mol |
| nichtaktiviertes Kraftlignin plus aktiviertes Lignin inkubiert mit Laccase | 11.000 g/Mol |

### BEISPIEL 3

Eine Ligninsuspension bestehend aus:
80 ml McIlvain Puffer pH 4,5
16,5 g Kraftlignin
4 g Ligninsulfonat
wurde mit konzentrierter Laccase auf eine Endkonzentration von 800 U/ml eingestellt und mit Preßluft für 3 h belüftet und gerührt. Die so erhaltene Lösung wurde auf Baumwollgewebe aufgebracht, wie es für die Herstellung von thermoplastischen Verbundwerkstoffen aus nachwachsenden Rohstoffen üblicherweise verwendet wird, und an der Luft getrocknet. Die nachfolgende Untersuchung des Produkts ergab einen Beleimungsgrad von 42 % atro Lignin bezogen auf atro Faser.

Um den Bindungsgrad des aufgebrachten Lignins zu ermitteln, wurde das beschichtete Gewebe für drei Stunden in Wasser bzw. in 0,1 m NaOH inkubiert und anschließend die Menge an gelöstem Lignin bestimmt. Durch die Wasserbehandlung lösten sich 2 % (w/w), durch die Behandlung mit Alkali 30 % (w/w) des aufgebrachten Lignins von der Baumwollfaser wieder ab.

### BEISPIEL 4

Nach dem in Beispiel 3 angegebenen Verfahren beschichtete Baumwollfaser wurde im Hochvakuum mit Gold besputtert und in einem Raster Elektronenmikroskop untersucht.

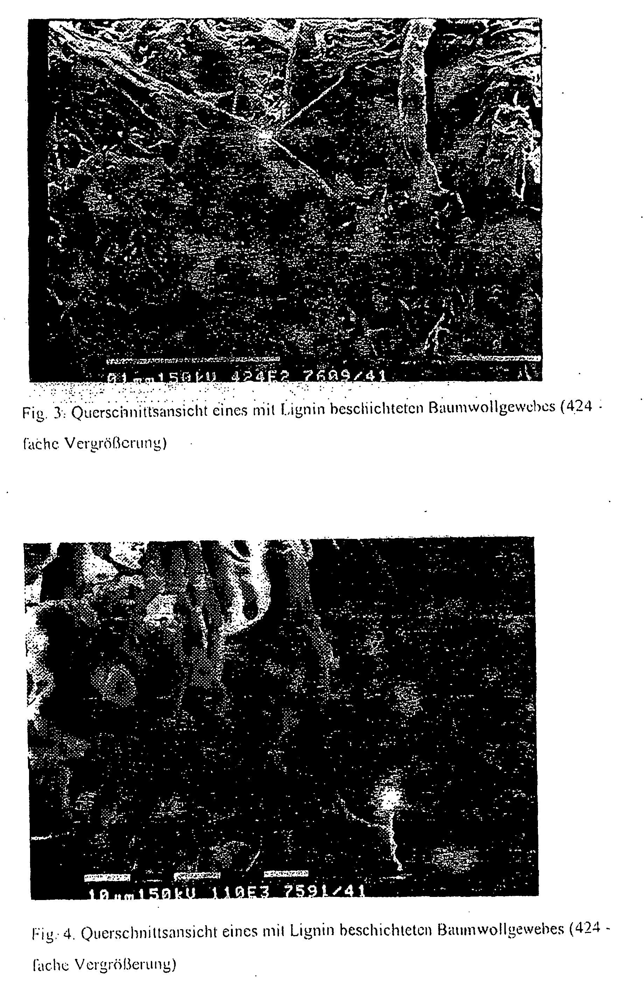

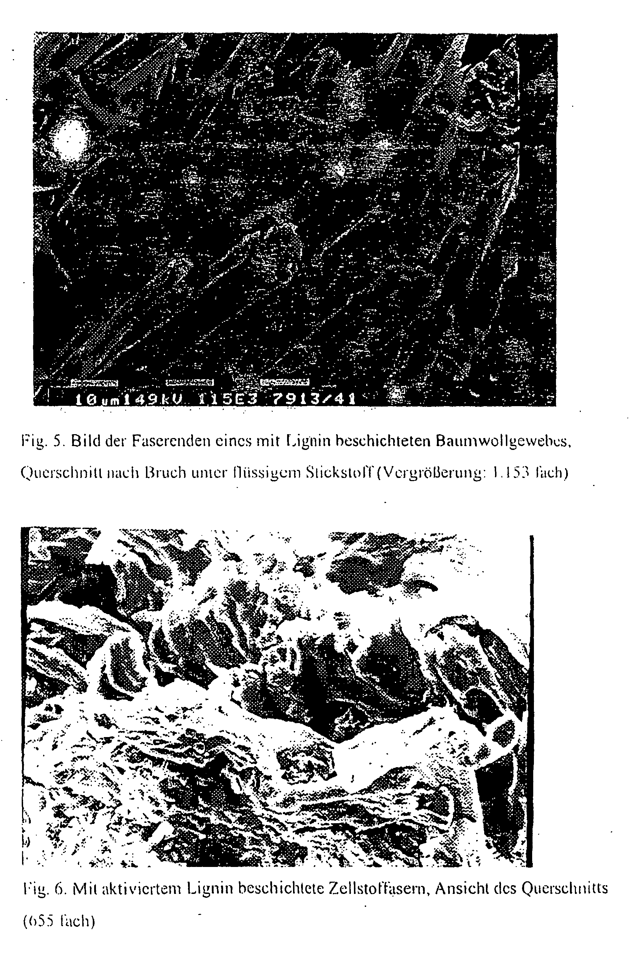

Figur 3, 4 und 5 zeigen, daß eine innige Verbindung zwischen der Beschichtung und der Faser erkennbar ist, ohne daß dabei eine Übergangszone sichtbar wird. Dies zeigt, daß die Beschichtung auf eine echte kovalente Bindung zwischen dem Lignin und der Faseroberfläche zurückzuführen ist.

### BEISPIEL 5:

20% Ligninleim bestehend aus:
80 ml McIlvain Puffer pH 4,5
16,5 g Kraftlignin
4 g Ligninsulfonat
wurden mit konzentrierter Laccase auf eine Endkonzentration von 800 U/ml eingestellt und mit Preßluft für 3 h belüftet und gerührt. Nach 3 h wurde die Lösung mit einer Laccaselösung (800 U/ml in Puffer pH 4,5) im Verhältnis 1 + 4 verdünnt und beidseitig auf Filterpapier aufgebracht.

Die behandelten Papiere wurden über Nacht an der Luft getrocknet. Die Papiere hatten einen Beschichtungsgrad von 9 % atro Lignin bezogen auf atro Papier.

Danach wurde die Festigkeit der Beschichtung gegenüber Wasser und 0,1 M NaOH ermittelt und die Wasseraufnahme bestimmt.

Eine dreistündige Inkubation in Wasser bewirkte eine Ablösung des Lignins um 3 % der aufgetragenen Menge, eine gleich lange Inkubation in 0,1 N NaOH löste 31 % des Lignins von der Papieroberfläche ab.

Die beschichteten Papiere hatten im Vergleich zu den unbeschichteten Kontrollen eine um 30 niedrigere Wasseraufnahme. Die Wasserreißfestigkeit war deutlich verbessert: während das unbehandelte Papier sich in einzelne Fasern auflöste, war das beschichtete Papier noch völlig intakt.

### BEISPIEL 6

Nach dem in Beispiel 3 angegebenen Verfahren beschichtetes Papier wurde im Hochvakuum mit Gold besputtert und in einem Raster Elektronenmikroskop untersucht.

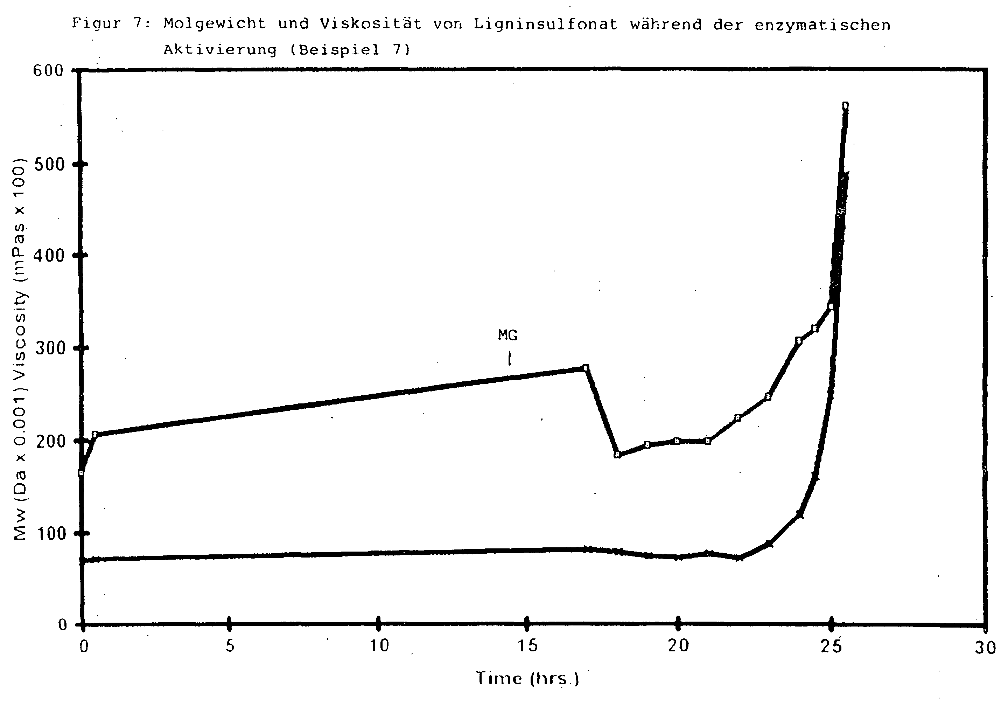

Fig. 6 läßt eine innige Verbindung zwischen der Beschichtung und der Papierfaser erkennen, ohne daß dabei eine Übergangszone sichtbar wird. Dies zeigt daß die Beschichtung auf eine echte kovalente Bindung zwischen dem Lignin und der Faser zurückzuführen ist.

### BEISPIEL 7:

Eine Lösung bestehend aus 80 ml McIlvain Puffer pH 4,5 und 20 g Ligninsulfonat wurde mit konzentrierter Laccase auf eine Endkonzentration von 800 U/ml eingestellt und für 25 h bei 37 °C im Wasserbad geschüttelt. Während der letzten Stunden stiegen sowohl das Molekulargewicht (gemessen in der HPLC) als auch die Viskosität der Lösung steil an (Fig. 7). Das so erhaltene Produkt war in Wasser, 0,1 m NaOH und in den üblichen organischen Lösungsmitteln wie Ethanol, Ether, Aceton oder Ethylacetat unlöslich.

## Patentansprüche

1. Zwischenprodukt für die Herstellung von Polymerisaten aus Ligninderivaten aus der Zellstoffindustrie, mit einem Signal für Phenoxyradikale im ESR-Spektrum, dessen Intensität mehr als das Fünffache des Signals des als Ausgangsprodukt dienenden Ligninderivats beträgt, hergestellt durch Behandlung des Ligninderivats mit dem phenoloxidierenden Enzym Laccase in Gegenwart von Oxidationsmitteln, wobei die Ligninderivate
a) länger als 3 Std. in Anwesenheit von Luft als Oxidationsmittel oder
b) länger als 10 min unter Durchleiten von Luft oder Sauerstoff als Oxidationsmittel der Enzymbehandlung unterworfen werden.

2. Zwischenprodukt nach Anspruch 1., **dadurch gekennzeichnet, daß** die Ligninderivate Kraftlignin oder Ligninsulfonat sind.

3. Zwischenprodukt nach Anspruch 1., **dadurch gekennzeichnet, daß** die Enzymbehandlung nach Stufe a) länger als 15 Stunden durchgeführt wird.

4. Zwischenprodukt nach Anspruch 1., **dadurch gekennzeichnet, daß** die Enzymbehandlung nach Stufe b) länger als 30 min. durchgeführt wird.

5. Zwischenprodukt nach Anspruch 1., **dadurch gekennzeichnet, daß** bei der Enzymbehandlung nach Stufe b) Druckluft oder Sauerstoff unter Druck eingeleitet wird.

6. Verwendung des Zwischenprodukts der Ansprüche 1 bis zur Herstellung von Polymerisaten von Ligninderivaten aus der Zellstoffindustrie.

7. Verwendung nach Anspruch 6., zur Herstellung von von hochaktiven Reagentien für die Herstellung von faserverstärkten duroplastischen Verbundwerkstoffen aus pflanzlichen Fasern.

8. Verwendung nach Anspruch 6., zur Herstellung von wasserfesten Papieren und Pappen.

9. Verwendung nach Anspruch 6., zur Herstellung von hochaktiven Reagentien für die Herstellung von Duroplasten aus Liguinderivaten.

10. Verfahren zur Herstellung von Polymerisaten aus Ligninderivaten aus der Zellstoffindustrie durch Behandlung der Ligninderivate mit phenoloxidierenden, Enzymen in Gegenwart von Oxidationsmitteln, **dadurch gekennzeichnet, daß** die Ligninderivate
a) länger als 3 Std in Anwesenheit von Luft der Enzymbehandlung unterworfen, oder
b) länger als 10 min unter Durchleitung von Luft oder Sauerstoff der Enzymbehandlung unterworfen werden.

11. Verfahren nach Anspruch 10., **dadurch gekennzeichnet, daß** die Ligninderivate Kraftlignin oder Ligninsulfonat sind.

12. Verfahren nach Anspruch 10., **dadurch gekennzeichnet, daß** die phenoloxidierenden Enzyme Phenoloxidase, Laccase sind.

13. Verfahren nach Anspruch 10, **dadurch gekennzeichnet, daß** die Enzymbehandlung nach Stufe a) länger als 15 Stunden durchgeführt wird.

14. Verfahren nach Anspruch 10., **dadurch gekennzeichnet, daß** die Enzymbehandlung nach Stufe b) länger als 30 min. durchgeführt wird.

15. Verfahren nach Anspruch 10., **dadurch gekennzeichnet, daß** bei der Enzymbehandlung nach Stufe b) Druckluft oder Sauerstoff unter Druck eingeleitet wird.

16. Verwendung der Erzeugnisse des Verfahrens der Ansprüche 10. bis 15. zur Herstellung von Polymerisaten von Ligninderivaten aus der Zellstoffindustrie.

17. Verwendung nach Anspruch 16. zur Herstellung von von hochaktiven Reagentien für die Herstellung von faserverstärkten duroplastischen Verbundwerkstoffen aus pflanzlichen Fasern.

18. Verwendung nach Anspruch 16. zur Herstellung von wasserfesten Papieren und Pappen.

19. Verwendung nach Anspruch 16. zur Herstellung von hochaktiven Reagentien für die Herstellung von Duroplasten aus Ligninderivaten.

## Claims

1. Intermediate product for the production of polymers from lignin derivates from cellulose industry, with a signal of phenoxy radicals in the ESR spectrum, whose intensity corresponds to more than five times the signal of the lignin derivates serving as starting product, manufactured by treating the lignin derivate with the phenol-oxidising enzyme laccase in the presence of oxidants, with the lignin derivates being subjected to an enzymatic treatment
(a) for more than 3 hours in the presence of air as oxidant or
(b) for more than 10 minutes when air or oxygen is passed therethrough as oxidant.

2. Intermediate product according to Claim 1, **characterised in that** said lignin derivates are kraft lignin or lignin sulphonate

3. Intermediate product according to Claim 1, **characterised in that** said enzymatic treatment according to stage (a) is carried out for more than 15 hours.

4. Intermediate product according to Claim 1, **characterised in that** said enzymatic treatment according to stage (a) is carried out for more than 30 minutes.

5. Intermediate product according to Claim 1, **characterised in that** compreesed air or pressurised oxygen is introduced in the enzymatic treatment according to stage (b).

6. Use of the intermediate product according to Claims 1 to 5 for the production of polymers of lignin derivates from cellulose industry.

7. Use according to Claim 6 for the production of highly reactive reagents for the manufacture of fibre-reinforced thermosetting composite materials made of vegetable fibres.

8. Use according to Claim 6 for the manufacture of water-resistant papers and cardboards.

9. Use according to Claim 6 for the production of highly reactive reagents for the thermosetting materials of lignin derivates.

10. Method of manufacturing polymers from lignin derivates from cellulose industry by treating the lignin derivates with phenol-oxidising enzymes in the presence of oxidants,
**characterised in that** said lignin derivates are
(a) subjected to the enzymatic treatment in the presence of air for more than 3 hours,
or
(b) are subjected to the enzymatic treatment for more than 10 minutes, with air or oxygen being passed therethrough.

11. Method according to Claim 10, **characterised in that** said lignin derivates are kraft lignin or lignin sulphonate.

12. Method according to Claim 10, **characterised in that** the phenol-oxidising enzymes are phenol oxidase, laccase.

13. Method according to Claim 10, **characterised in that** the enzyme treatment according to stage (a) is carried out for more than 15 hours.

14. Method according to Claim 10, **characterised in that** the enzyme treatment according to stage (b) is carried out for more than 30 minutes.

15. Method according to Claim 10, **characterised in that** compressed air or pressurised oxygen is introduced during the enzyme treatment according to stage (b).

16. Use of the products obtained by the method according to Claims 10 to 15 for the production of polymers of lignin derivates from cellulose industry.

17. Use according to Claim 16 for the production of highly active reagents for the manufacture of fibre-reinforced thermosetting composite materials made of vegetable fibres.

18. Use according to Claim 16 for the manufacture of water-resistant papers and cardboards.

19. Use according to Claim 16 for the production of highly active reagents for the manufacture of thermosetting materials from lignin derivates.

## Revendications

1. Produit intermédiaire pour la production des polymères à partir des dérivatives de la lignine à origine de l'industrie de la celluslose technique, à un signal des radicaux phenoxy dans le spectre ESR, dont l'intensité corresponds à plus de cinq fois le signal des dérivatives de la lignine, qui servent comme matière première, produits en traitant le dérivative de la lignine par l'enzyme laccase oxydant le phénol, en présence des oxydants, auxdits dérivatives de la lignine étant assujettis à un traitement enzymatique
(a) pour plus de 3 heures en présence de l'air en tant que l'oxydant
ou
(b) pour plus de 10 minutes quand de l'air ou de l'oxygène est y passé en tant que l'oxydant.

2. Produit intermédiaire selon la revendication 1, **caractérisé en ce que** lesdits dérivatives de la lignine sont de la lignine kraft ou du sulfonât de la lignine.

3. Produit intermédiaire selon la revendication 1, **caractérisé en ce que** ledit traitement enzymatique selon l'étape (a) se fait pour plus de 15 heures.

4. Produit intermédiaire selon la revendication 1, **caractérisé en ce que** ledit traitement enzymatique selon l'étape (b) se fait pour plus de 30 minutes.

5. Produit intermédiaire selon la revendication 1, **caractérisé en ce que** de l'air comprimé ou de l'oxygène sous pression est introduit au cours du traitement enzymatique selon l'étape (b).

6. Emploi du produit intermédiaire selon les revendications 1 à 5 pour la production des polymères des dérivatives de la lignine à origine dans l'industrie de la cellulose technique.

7. Emploi selon la revendication 6 pour la production des réactifs à haute réactivité pour la fabrication des matériaux composites thermodurcissables, renforcés par des fibres, faits en fibres végétales.

8. Emploi selon la revendication 6 pour la fabrication des papiers et cartons hydrorésistants.

9. Emploi selon la revendication 6 pour la production de des réactifs à haute réactivité pour les résines thermodurcissables des dérivatives de la lignine.

10. Procédé à produire des polymères à partir des dérivatives de la lignine à l'origine dans l'industrie de la cellulose technique, en traitant les dérivatives de la lignine par des enzymes oxydant le phénol, en présence des oxydants,
**caractérisé en ce que** lesdits dérivatives de la lignine sont
(a) assujettis au traitement enzymatique en présence de l'air pour plus de 3 heures,
ou
(b) sont assujettis au traitement enzymatique pour plus de 10 minutes, en y passant de l'air ou de l'oxygène.

11. Procédé selon la revendication 10, **caractérisé en ce que** lesdits dérivatives de la lignine sont de la lignine kraft ou du sulfonât de la lignine.

12. Procédé selon la revendication 10, **caractérisé en ce que** les enzymes oxydant le phénol sont de l'oxydase de phénol, de la laccase.

13. Procédé selon la revendication 10, **caractérisé en ce que** le traitement enzymatique selon l'étape (a) se fait pour plus de 15 heures.

14. Procédé selon la revendication 10, **caractérisé en ce que** le traitement enzymatique selon l'étape (b) se fait pour plus de 30 minutes.

15. Procédé selon la revendication 10, **caractérisé en ce que** de l'air comprimé ou de l'oxygène sous pression est introduit au cours du traitement enzymatique selon l'étape (b).

16. Emploi des produits obtenus par le procédé selon les revendications 10 à 15 pour la production des polymères des dérivatives de la lignine à l'origine dans l'industrie de la cellulose technique.

17. Emploi selon la revendication 16 pour la production des réactifs à haute réactivité pour la fabrication des matériaux composites thermodurcissables, renforcés par des fibres, qui sont faits en fibres végétales.

18. Emploi selon la revendication 16 pour la fabrication des papiers et cartons hydrorésistants.

19. Emploi selon la revendication 16 pour la production des réactifs à haute réactivité pour la fabrication des résines thermodurcissables à partir des dérivatives de la lignine.
